# EUROPÄISCHE PATENTSCHRIFT

(11) **EP 1 097 140 B1**
(45) Veröffentlichungstag und Bekanntmachung des Hinweises auf die Patenterteilung: **03.03.2004**
(21) Anmeldenummer: 99934669.5
(22) Anmeldetag: 10.07.1999
(51) Int. Cl.: C07D 233/70, C07D 233/68, C07D 233/54, A61K 31/415

(54) **IMIDAZOLDERIVATE MIT BIPHENYLSULFONYLSUBSTITUTION, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
BIPHENYLSULFONYL-SUBSTITUTED IMIDAZOLE DERIVATIVES, THEIR PREPARATION PROCESS, THEIR USE AS A DRUG OR DIAGNOSTIC AGENT AND DRUG CONTAINING THEM
DERIVES D'IMIDAZOLE A SUBSTITUTION BIPHENYLSULFONYLE, LEUR PROCEDE DE PREPARATION, LEUR UTILISATION COMME MEDICAMENT OU AGENT DIAGNOSTIQUE, AINSI QUE MEDICAMENT LES CONTENANT

(30) Priorität: 18.07.1998 DE 19832429
(43) Veröffentlichungstag der Anmeldung: 09.05.2001
(73) Patentinhaber: Aventis Pharma Deutschland GmbH, 65929 Frankfurt am Main (DE)
(72) Erfinder: KLEEMAN, Heinz-Werner, D-65474 Bischofsheim (DE); LANG, Hans, Jochen, D-65719 Hofheim (DE); SCHWARK, Jan-Robert, D-65779 Kelkheim (DE); WEICHERT, Andreas, D-63329 Egelsbach (DE); PETRY, Stefan, D-65929 Frankfurt (DE)
(86) Internationale Anmeldenummer: PCT/EP1999/004886
(87) Internationale Veröffentlichungsnummer: WO 2000/003994

(56) Entgegenhaltungen:
- EP-A- 0 479 479
- EP-A- 0 503 838
- EP-A- 0 855 392
- WO-A-92/20662
- WO-A-94/03435
- WO-A-95/23791
- US-A- 5 391 732
- US-A- 5 482 957
- US-A- 5 604 251

## Beschreibung

Imidazolderivate mit Biphenylsulfonylsubstitution, Verfahren zu ihrer Herstellung, ihre Verwendung als Medikament oder Diagnostikum sowie sie enthaltendes Medikament

Die Erfindung betrifft Verbindungen der Formel I, 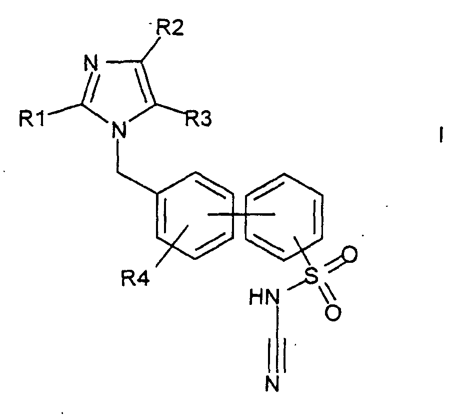 in welcher die Symbole folgende Bedeutung haben:
R(1) -CₐH₂ₐ-Phenyl, wobei der Phenylteil unsubstituiert oder substituiert ist mit 1 oder 2 gleichen oder verschiedenen Resten aus der Reihe F, Cl, Br, CF₃, Methyl, Methoxy, Hydroxy oder NR(8)R(9); R(8) und R(9) unabhängig voneinander Wasserstoff oder Methyl;
   a Null oder 1;
R(2) F, Cl, Br oder I; oder O(R7); R(7) Alkyl mit 1, 2, 3 oder 4 C-Atomen;
R(3) CO-R(6);
   R(6) Wasserstoff;
R(4) SO₂R(16) mit R(16) gleich Alkyl mit 1, 2, 3 oder 4 C-Atomen;
sowie deren physiologisch verträglichen Salze.

Bevorzugt sind insbesondere Verbindungen der Formel I, in denen R(4) SO₂R(16) mit R(16) gleich Alkyl mit 1, 2, 3 oder 4 C-Atomen, insbesondere Methyl bedeutet und R(1), R(2) und R(3) wie oben definiert sind, sowie deren physiologisch verträglichen Salze.

Bevorzugt sind desweiteren Verbindungen der Formel I, in denen die Reste R(1 ), R(2), R(3) und R(4) wie oben definiert sind und der Biphenylsubstituent wie in Formel la oder lb, bevorzugt la verknüpft ist, 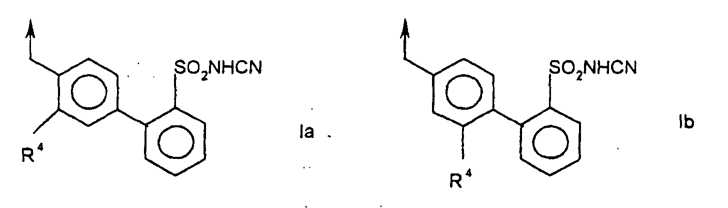 sowie deren physiologisch verträglichen Salze.

Alkylreste und Alkylenreste können geradkettig oder verzweigt sein. Dies gilt auch für die Alkylenreste der Formeln CₐH₂ₐ, C_{b}H_{2b}, C_{d}H_{2d}, C_{g}H_{2g}, und CₗH₂ₗ. Alkylreste und Alkylenreste können auch geradkettig oder verzweigt sein, wenn sie substituiert sind oder in anderen Resten enthalten sind, z. B. in einem Alkoxyrest oder in einem Alkylmercaptorest oder in einem fluorierten Alkylrest.

Beipiele für Alkylreste mit 1, 2, 3, 4, 5, 6, 7 oder 8 C-Atomen sind: Methyl, Ethyl, n-Propyl, n-Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,4-Butylen, 1,5-Pentylen, 2,2-Dimethyl-1,3-propylen, 1,6-Hexylen, usw. sind Beispiele für Alkylenreste.

Monosubstituierte Phenylreste können in der 2-, der 3- oder der 4-Position substituiert sein, disubstituierte in der 2,3-, 2,4-, 2,5-, 2,6-, 3,4- oder 3,5-Position, trisubstituierte in der 2,3,4-, 2,3,5-, 2,3,6- 2,4,5-, 2,4,6- oder 3,4,5-Position.
Entsprechendes gilt sinngemäß analog auch für die N-haltigen Heterocylen oder den Thiophenrest.
Bei Di- oder Trisubstitution eines Restes können die Substituenten gleich oder verschieden sein.

Enthalten die Verbindungen der Formel I eine oder mehrere saure oder basische Gruppen bzw. einen oder mehrere basische Heterocyclen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.
Unter physiologisch verträglichen Salzen von Verbindungen der Formel (I) sind beispielsweise auch organische und anorganische Salze zu verstehen , wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z. B. Essigsäure, Zitronensäure, Benzoesäure, Maleinsäure, Fumarsäure, Weinsäure und p-Toluolsulfonsäure bevorzugt.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. sind also in enantiomerenreiner Form, sowohl als links- als auch als rechtsdrehende Antipoden, und auch in Form von Mischungen der beiden Enantiomeren in unterschiedlichen Verhältnissen oder in Form von Räcematen Gegenstand der Erfindung. Bei Vorliegen einer cis/trans-Isomerie sind sowohl die cis-Form als auch die trans-Form und Gemische dieser Formen Gegenstand der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfaßt die vorliegende Erfindung auch alle tautomeren Formen der Verbindungen der Formel I.

Die Erfindung betrifft auch ein Verfahren zur Herstellung der Verbindungen der Formel I, sowie deren physiologisch verträglicher Salze, das dadurch gekennzeichnet ist, daß man eine Verbindung der Formel II 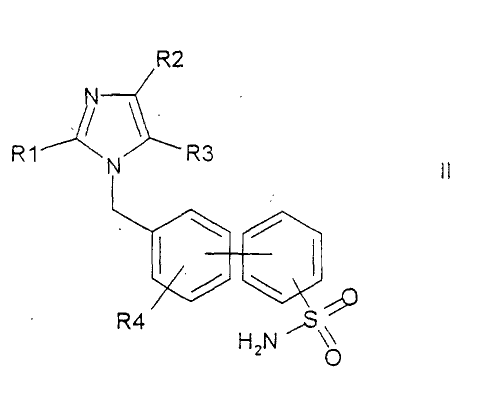 worin die Reste wie oben definiert sind und die analog zu J. Med. Chem. 1995, 38, 2357 in an sich bekannter Weise hergestellt werden können, mit Bromcyan umsetzt.

Die Reaktion erfolgt vorteilhaft in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur von 0°C bis zu dem Siedepunkt des verwendeten Lösungsmittels in Frage, bevorzugt ist eine Temperatur von 60°C bis 120°C.

Die Einführung des Substituenten R(4) erfolgt vorteilhaft auf der Stufe des Toluolderivats III, 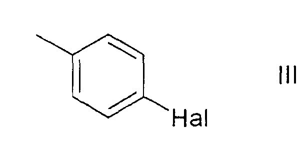 wobei Hal eine mit der Suzuki-Reaktion vereinbare Abgangsgruppe, bevorzugt Brom oder lod bedeutet. Die Einführung eines SO₂-Alkyl-Restes über Chlorsulfonierung ist beispielhaft in J. Med. Chem. 1997, 40, 2017 oder J. Org. Chem. (1991), 56(16), 4974-6 beschrieben.

Alle Reaktionen zur Synthese der Verbindungen der Formel I sind dem Fachmann an sich wohlbekannt und können unter Standardbedingungen nach oder analog zu Literaturvorschriften durchgeführt werden, wie sie zum Beispiel in Houben-Weyl, Methoden der organischen Chemie, Thieme-Verlag, Stuttgart, oder Organic Reactions, John Wiley & Sons, New York, beschrieben sind. Je nach den Gegebenheiten des Einzelfalls kann es bei der Synthese der Verbindungen der Formel I zur Vermeidung von Nebenreaktionen auch vorteilhaft oder notwendig sein, bestimmte funktionelle Gruppen vorübergehend durch die Enführung von Schutzgruppen zu blockierenn und später dann wieder freizusetzen oer funktionelle Gruppen zunächst in Form von Vorstufen einzusetzen, aus denen in einem späteren Schritt dann die gewünschte funktionelle Gruppe erzeugt wird. Solche Synthesestrategien und die für den Einzelfall geeigneten Schutzgruppen oder Vorstufen sind dem Fachmann bekannt. Die erhaltenen Verbindungen der Formel I können gegebenenfalls nach üblichen Reinigungsmethoden, zum Beispiel durch Umkristallisation oder Chromatographie, gereinigt werden. Die Ausgangsverbindungen für die Herstellung der Verbindungen der Formel I sind käuflich erhältlich oder können nach oder analog zu Literaturvorschriften hergestellt werden.

Desweiteren betrifft die Erfindung die Verwendung einer Verbindung der Formel I und/oder eines physiologisch verträglichen Salzes davon zur Herstellung eines Medikaments zur Behandlung oder Prophylaxe von durch ischämische Zustände hervorgerufenen Krankheiten;
sowie die Verwendung einer Verbindung der Formel I und/oder eines physiologisch verträglichen Salzes davon zur Herstellung eines Medikaments zur Behandlung oder Prophylaxe des Herzinfarkts;
sowie die Verwendung einer Verbindung der Formel I und/oder eines physiologisch verträglichen Salzes davon zur Herstellung eines Medikaments zur Behandlung oder Prophylaxe der Angina Pectoris;
sowie die Verwendung einer Verbindung der Formel I und/oder eines physiologisch verträglichen Salzes davon zur Herstellung eines Medikaments zur Behandlung oder Prophylaxe von ischämischen Zuständen des Herzens;
sowie die Verwendung einer Verbindung der Formel I und/oder eines physiologisch verträglichen Salzes davon zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I und/oder eines physiologisch verträglichen Salzes davon zur Herstellung eines Medikaments zur Behandlung oder Prophylaxe von ischämischen Zuständen peripherer Organe und Gliedmaßen;
sowie die Verwendung einer Verbindung der Formel I und/oder eines physiologisch verträglichen Salzes davon zur Herstellung eines Medikaments zur Behandlung von Schockzuständen;
sowie die Verwendung einer Verbindung der Formel I und/oder eines physiologisch verträglichen Salzes davon zur Herstellung eines Medikaments zum Einsatz bei chirurgischen Operationen und Organtransplantationen;
sowie die Verwendung einer Verbindung der Formel I und/oder eines physiologisch verträglichen Salzes davon zur Herstellung eines Medikaments zur Konservierung und Lagerung von Transplantaten für chirurgische Maßnahmen;
sowie die Verwendung einer Verbindung der Formel I und/oder eines physiologisch verträglichen Salzes davon zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I und/oder eines physiologisch verträglichen Salzes davon zur Herstellung eines Medikaments zur Behandlung von gestörtem Atemantrieb;
sowie eine pharmazeutische Zubereitung, gekennzeichnet durch einen wirksamen Gehalt einer Verbindung der Formel I und/oder eines physiologisch verträglichen Salzes davon.

Die erfindungsgemäßen Verbindungen der Formel I eignen sich als Inhibitoren des Natrium-abhängigen Bicarbonat/Chlorid-Austauschers (NCBE) bzw. des Natrium/Bicarbonat-Symporters.

Den erfindungsgemäßen Verbindungen der Formel I ähnliche Verbindungen sind aus den US-Patentschriften 5,482,957 und 5,604,251 bekannt. Sie weisen jedoch nicht die nach der Erfindung stets vorhandene Sulfonylcyanamid-Seitenkette auf. Imidazolderivate als Angiotensin II-antagonisten werden auch in WO9523792, WO9523791, US 5391732, EP-A 648763 beschrieben. Die bekannten Verbindungen sind Angiotensin II-Rezeptor-Antagonisten des Subtyps AT1, welche Wirkung bei den erfindungsgemäßen Verbindungen I nicht oder nur im geringen Ausmaß vorhanden ist.

In der älteren europäischen Patentanmeldung EP-A 855 392 werden Imidazolderivate mit einer Biphenylsulfonylcyanamid-Seitenkette als NCBE-Inhibitoren vorgeschlagen, unter deren allgemeinen Formel die erfindungsgemäßen Verbindungen fallen.

Die in der vorliegenden Erfindung beschriebenen Imidazolderivate mit BiphenylsuLfonylcyanamid-Seitenkette weisen einen speziellen Substituenten R(4) am Biphenylgerüst auf und zeichnen sich durch eine hohe Wirksamkeit in der Inhibition des zellulären Na⁺-abhängigen Bicarbonat/Chlorid-Austauschs sowie einer verbesserten Bioverfügbarkeit aus.

Die erfindungsgemäßen Verbindungen der Formel (I) zeigen sehr gute antiarrhythmische Eigenschaften, wie sie beispielsweise für die Behandlung von Krankheiten wichtig sind, welche bei Sauerstoffmangelerscheinungen auftreten. Die Verbindungen der Formel (I) sind infolge ihrer pharmakologischen Eigenschaften als antiarrhythmische Arzneimittel mit cardioprotektiver Komponente zur Infarktprophylaxe und der Infarktbehandlung sowie zur Behandlung der Angina Pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern.

Wegen ihrer schützenden Wirkungen gegen pathologische hypoxische und ischämische Situationen können die erfindungsgemäßen Verbindungen der Formel (I) infolge Inhibition des zellulären Na⁺-abhängigen Cl⁻/HCO₃⁻-Austauschmechanismus (NCBE) bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie schützen akut oder chronisch sauerstoffmangel-versorgte Organe durch Verringerung oder Verhinderung ischämisch induzierter Schäden und eignen sich somit als Arzneimittel beispielsweise bei Thrombosen, Gefäßspasmen, Atherosklerose oder bei operativen Eingriffen (z.B. bei Organ-Transplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen.

Die Verbindungen der Formel (I) sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel (I) ebenfalls zur Behandlung von Formen des Schocks, wie beispielsweise des allergischen, cardiogenen, hypovolämischen und des bakteriellen Schocks.

Darüberhinaus zeichnen sich die erfindungsgemäßen Verbindungen der Formel (I) durch starke inhibierende Wirkung auf die Proliferationen von Zellen, beispielsweise der Fibroblasten-Zellproliferation und der Proliferation der glatten Gefäßmuskelzellen und der Mesangiumzellen aus. Deshalb kommen die Verbindungen der Formel (I) als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und/oder -hyperplasien, insbesondere bei Prostatahyperplasie bzw. Prostatahypertrophie verwendet werden.

Es wurde gefunden, daß Inhibitoren des Na⁺-abhängigen Cl⁻/HCO₃⁻ -Austauschers (NCBE-Inhibitoren) bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren. Eine Verbesserung des Atemantriebes durch Stimulation der Chemorezeptoren mit Substanzen, die den Na⁺-abhängigen Cl⁻/HCO₃⁻ -Austausch hemmen, führt zu einer Verbesserung der Atmung bei folgenden klinischen Zuständen und Krankheiten: Gestörter zentraler Atemantrieb (z.B. zentrale Schlafapnoen, plötzlicher Kindstod, postoperative Hypoxie), muskulär-bedingte Atemstörungen, Atemstörungen nach Langzeitbeatmung, Atemstörungen bei Adapation im Hochgebirge, obstruktive und gemischte Form der Schlafapnoen, akute und chronische Lungenkrankheiten mit Hypoxie und Hyperkapnie.

Die erfindungsgemäßen Verbindungen der Formel I und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I und ihre physiologisch verträglichen Salze zur Anwendung als Arzneimittel, ihre Verwendung in der Therapie und Prophylaxe der genannten Krankheitsbilder und ihre Herstellung von Medikamenten dafür. Weiterhin sind Gegenstand der vorliegenden Erfindung pharmazeutische Zubereitungen, die als aktiven Bestandteil eine wirksame Dosis mindestens einer Verbindung der Formel I und/oder eines physiologisch verträglichen Salzes davon neben üblichen pharmazeutisch einwandfreien Träger- und Hilfsstoffen enthalten. Die pharmazeutischen Zubereitungen enthalten normalerweise 0,1 bis 99 Gewichtsprozent, bevorzugt 0,5 bis 95 Gewichtsprozent der Verbindungen der Formel I und/oder ihrer physiologisch verträglichen Salze. Die Herstellung der pharmazeutischen Zubereitungen kann in an sich bekannter Weise erfolgen. Dazu werden die Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die eine Verbindung der Formel (I) und/oder ihre physiologisch verträglichen Salze enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken-als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs einer Verbindung der Formel (I) und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft desweiteren die Kombination von a) NCBE-Inhibitoren der Formel I und/oder deren physiologisch verträglichen Salze mit NHE-Inhibitoren und/oder deren physiologisch verträglichen Salze; b) NCBE-Inhibitoren der Formel I und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren der Formel I und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., DIURETICS, Chemistry, Pharmacology and Medicine, J. WILEY & Sons (1983), 303 - 341 beschrieben sind oder um die in DE19737224.4 aufgeführten NHE-Inhibitoren.

Geeignete NHE-Inhibitoren sind beispielsweise auch Benzoylguanidine, wie sie in US 5292755, US 5373024, US 5364868, US 5591754, US 5516805, US 5559153, US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, EP-A 814077, EP-A 869116 beschrieben sind; ortho-substituierte Benzoylguanidine, wie sie in EP-A 556673, EP-A 791577, EP-A 794172 beschrieben sind; ortho-amino-substituierte Benzoylguanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkyl- bzw. Alkenyl-carbonsäure-Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind; Indenoylguanidine, wie sie in EP-A 738712 beschrieben sind; Benzyloxycarbonyl-guanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäurediguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäureguanidine, wie sie in EP-A 790245 beschrieben sind; Bis-ortho-substituierte Benzoylguanidine, wie sie in EP-A 810207 beschrieben sind; Substituierte 1- oder 2-Naphthylguanidine, wie sie in EP-A 810205 und EP-A 810206 beschrieben sind; Indanylidin-acetylguanidine, wie sie in EP-A 837055 beschrieben sind; Phenylsubstituierte Alkenylcarbonsäure-guanidine, wie sie in EP-A 825178 beschrieben sind; Aminopiperidyl-Benzoylguanidine, wie sie in EP-A 667341 beschrieben sind; Heterocycloxy-Benzylguanidine, wie sie in EP-A 694537 beschrieben sind; ortho-substituierte Benzoylguanidine, wie sie in EP-A 704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie EP-A 708088 beschrieben sind; ortho-substituierte 5-Alkylsulfonyl-benzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyloder 3-Dihalophenyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonyl-benzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoylguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie in JP 9067332 beschrieben sind; Propenoylguanidine mit Indolsubstituenten, wie sie in JP 9067340 beschrieben sind; oder Heteroaryl-substituierte Acroylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am meisten bevorzugt ist Cariporid oder ein anderes physiologisch verträgliches Salz des N-(4-Isopropyl-3-methansulfonyl-benzoyl)-guanidin.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridin-aktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.
Ein Beispiel eines solchen besonders vorteilhaften Kombinationspartners mit NCBE-Inhibitoren sind Blocker des nicht-inaktivierenden-Natriumkanals (Veratridin-aktivierbarer Natriumkanal). Die Kombinationen eines NCBE-Inhibitors mit einem Blocker des nicht-inaktivierenden-Natriumkanals (Veratridin-aktivierbarer Natriumkanal) eignen sich zur Infarkt- und Re-infarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors mit einem Blocker des nicht-inaktivierenden-Natriumkanals infolge verstärkter Inhibierung des Na+ Einstroms in die Zelle als Arzneimittel zur Behandlung aller akuten oder chronischen, durch Ischämie ausgelösten Schäden oder dadurch primär oder sekundäre induzierter Krankheiten verwendet werden. Dies betrifft ihre Verwendung als Arzneimittel für operative Eingriffe, z. B. bei Organtransplantationen, wobei die Kombinationen eines NCBE-Inhibitors mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NCBE-Inhibitors mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren der Formel I und/oder deren physiologisch verträgliches Salze mit NHE-Inhibitoren und/oder einer zusätzlichen aktiven Substanz aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor der Formel I und/oder deren physiologisch verträgliches Salz und einen NHE-Inhibitor und/oder deren physiologisch verträglichen Salze; oder b) einen NCBE-Inhibitor der Formel I und/oder deren physiologisch verträgliches Salz und zusätzlich eine aktive Substanz aus einer anderen Herz-Kreislauf-Wirkstoffklasse und/oder deren physiologisch verträglichen Salze; oder c) einen NCBE-Inhibitor der Formel I und/oder deren physiologisch verträgliches Salz, einen NHE-Inhibitor und zusätzlich eine aktive Substanz aus einer anderen Herz-Kreislauf-Wirkstoffklasse, und/oder deren physiologisch verträglichen Salze.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf-Wirkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichstverhältnis vom NBCE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100 , vorzugsweise 1:0,1 bis 1:10.

Die erfindungsgemäßen Kombination und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschieden Wirkstoffkomponenten im Berreich von 0,001 bis 100 mg/kg/Tag.

### Liste der Abkürzungen:

- BCECF: 2',7'-Bis(2-carboxyethyl)-5,6,carboxyfluorescein
- CH₂Cl₂: Dichlormethan
- DCl: Desorption-Chemical Ionisation
- DMF: N,N-Dimethylformamid
- EE: Ethylacetat (EtOAc)
- ES: Elektrospray-lonisation
- FAB: Fast Atom Bombardment
- HEP: n-Heptan
- mp: Schmelzpunkt
- NCBE: Natriumabhängiger Chlorid/Bicarbonat-Austauscher
- NHE: Natrium/Wasserstoff-Austauscher
- RT: Raumtemperatur
- ZNS: Zentralnervensystem

### Allgemeine Vorschrift zur Herstellung von Sulfonylcyanamiden aus Sulfonamiden

Das Sulfonamid-Ausgangsmaterial wird in 10 ml/mmol wasserfreiem Acetonitril gelöst, 3 Mol-Äquivalente K₂CO₃ sowie ein Mol-Äquivalent einer 5 N Lösung von BrCN in Acetonitril zugetropft und bis zum vollständigen Umsatz unter Rückfluß erhitzt (typische Reaktionszeit 10 Minuten bis 6 Stunden). Das Reaktionsgemisch wird anschließend ohne weitere Aufarbeitung an Kieselgel chromatographiert.

### Beispiele:

### Beispiel 1:

### 4'-(5-Formyl-4-methoxy-2-phenyl-imidazol-1-ylmethyl)-3'-methansulfonyl-biphenyl-2-sulfonylcyanamid

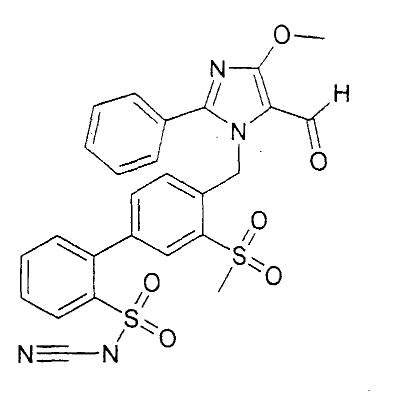

### a) 2-Brom-5-methyl-benzolsulfonyl chlorid

40 g 4-Bromtoluol werden unter Rühren langsam bei -10 °C in 250 ml Chlorsulfonsäure eingetragen. 30 Minuten wird bei dieser Temperatur nachgerührt, man läßt auf 0 °C erwärmen und gießt auf überschüssiges Eis. Das Produkt wird abgesaugt und mit wenig Wasser gewaschen. Über P₄O₁₀ wird im Vakuum getrocknet und man erhält 63 g eines farblosen Feststoffs, der direkt weiter umgesetzt wird.

### b) 2-Brom-5-methyl-benzolsulfinsäure

37.6 g Natriumsulfit werden in 500 ml Wasser gelöst und auf 70 ° C erwärmt. Bei dieser Temperatur werden portionsweise 62 g 2-Brom-5-methyl-benzolsulfonyl chlorid zugegeben. Hierbei wird gleichzeitig eine 10 N wäßrige NaOH-Lösung zugetropft, sodaß der pH-Wert der Lösung zwischen pH=9 und pH=10 gehalten wird. 1.5 Stunden wird bei 70 °C nachgerührt, die Lösung abfiltriert und anschließend im Eisbad mit einer gesättigten wäßrigen HCl-Lösung auf pH=0 gestellt. 30 Minuten wird nachgerührt, anschließend das Produkt abfiltriert, mit wenig Wasser nachgewaschen und getrocknet. Man erhält 49.6 g weißer Kristalle,
mp 120-122 °C. MS (ES) : 236 (M+H)⁺

### c) Natrium-2-brom-5-methyl-benzolsulfinat

49.6 g 2-Brom-5-methyl-benzolsulfinsäure werden in 400 ml Methanol gelöst und mit einer äquimolaren Menge NaOH in 50 ml Wasser versetzt. 3 Stunden wird bei RT nachgerührt, die Lösung abfiltriert und anschließend die Solventien im Vakuum entfernt. Zuletzt werden Wasserreste azeotrop mit 50 ml Toluol entfernt. Der feste Rückstand wird über P₄O₁₀ wird im Vakuum getrocknet und man erhält 54.0 g Produkt, mp 288-290 °C (unter Zersetzung).

### d) 1-Brom-2-methansulfonyl-4-methyl-benzol

54.0 g Natrium-2-brom-5-methyl-benzolsulfinat werden in 300 ml wasserfreiem DMF suspendiert und mit 45.7 ml Methyliodid versetzt. Dabei steigt die Temperatur der Lösung auf 50 °C an. 3 Stunden wird bei 50 °C nachgerührt und das DMF im Vakuum entfernt. Der Rückstand wird mit 500 ml Wasser verrührt, 1 Stunde bei 0 °C nachgerührt und abfiltriert. Das Produkt wird mit Wasser gewaschen, getrocknet und aus 400 ml HEP/250 ml EE mit Aktivkohle umkristallisiert. Man erhält 27.0 g farbloser Kristalle, mp 110-114 °C.
R_{f}(EE/HEP 1:4) = 0.09 MS (DCI) : 250 (M+H)⁺

### e) 1-Brom-4-brommethyl-2-methansulfonyl-benzol

9.9 g 1-Brom-2-methansulfonyl-4-methyl-benzol werden in 100 ml Chlorbenzol aufgenommen, 77 mg Benzoylperoxid sowie 7.1 g N-Bromsuccinimid zugegeben und 1 Stunde unter Rückfluß gekocht. Anschließend wird das Solvens im Vakuum entfernt, der Rückstand in 100 ml CH₂Cl₂ aufgenommen und 2 mal mit 50 ml einer gesättigten wäßrigen Na₂CO₃-Lösung und 1 mal mit 50 ml Wasser gewaschen. Über Na₂SO₄ wird getrocknet und das Solvens im Vakuum entfernt. Der Rückstand wird aus 80 ml HEP/30 ml EE umkristallisiert und man erhält 6.9 g eines blaßgelben Feststoffs, mp 120-124 °C.
R_{f}(EE/HEP 1:2) = 0.38 MS (DCI) : 329 (M+H)⁺

### f) 3-(4-Brom-3-methanesulfonyl-benzyl)-5-chlor-2-phenyl-3H-imidazol-4-carbaldehyd

1.0 g 5-Chloro-2-phenyl-3H-imidazol-4-carbaldehyd (Chem. Pharm. Bull. 1976, 24(5), 960), 1.6 g 1-Brom-4-brommethyl-2-methansulfonyl-benzol und 691 mg K₂CO₃ werden in 25 ml wasserfreiem DMF 18 Stunden bei RT gerührt. Das Reaktionsgemisch wird auf 300 ml einer halbgesättigten wäßrigen NaHCO₃-Lösung gegossen und 3 mal mit je 150 ml EE extrahiert. Über Na₂SO₄ wird getrocknet und das Solvens im Vakuum entfernt. Chromatographie an Kieselgel mit EE/HEP 1:2 liefert 1.2 g eines farblosen Öls.
R_{f}(EE/HEP 1:2) = 0.16 MS (FAB) : 454 (M+H)⁺

### g) 4'-(4-Chloro-5-formyl-2-phenyl-imidazol-1-ylmethyl)-3'-methansulfonyl-biphenyl-2-sulfonsäure-tert-butylamid

970 mg 3-(4-Brom-3-methanesulfonyl-benzyl)-5-chlor-2-phenyl-3H-imidazol-4-carbaldehyd, 660 mg N-tert.-Butyl-2-dihydroxyboran-2-yl-benzolsulfonamid (J. Med. Chem. 1997, 40, 547), 24 mg Pd(II)-acetat und 56 mg Triphenylphosphin werden in 13 ml Toluol und 3.5 ml Ethanol aufgenommen und 2.1 ml einer wäßrigen 2 M Na₂CO₃-Lösung zugesetzt. 105 Minuten wird unter Rückfluß gekocht, dann läßt man auf RT abkühlen und das Reaktionsgemisch wird in 200 ml einer halbgesätigten wäßrigen NaHCO₃-Lösung aufgenommen. 3 mal wird mit je 150 ml EE extrahiert, über Na₂SO₄ getrocknet und das Solvens im Vakuum entfernt. Chromatographie an Kieselgel mit EE/HEP 1:2 liefert 660 mg eines farblosen Öls.
R_{f}(EE/HEP 1:2) = 0.12 MS (ES) : 587 (M+H)⁺

### h) 4'-(4-Chloro-5-formyl-2-phenyl-imidazol-1-ylmethyl)-3'-methansulfonyl-biphenyl-2-sulfonamid

650 mg 4'-(4-Chloro-5-formyl-2-phenyl-imidazol-1-ylmethyl)-3'-methansulfonylbiphenyl-2-sulfonsäure-tert-butylamid werden in 5.6 ml Trifluoressigsäure gelöst und 133 µl Anisol zugespritzt. 8 Stunden wird bei RT gerührt, anschließend die flüchtigen Bestandteile im Vakuum entfernt. 3 mal wird der Rückstand erneut in 20 ml Wasser aufgenommen und das Wasser anschließend im Vakuum entfernt. Schließlich wird der Rückstand noch 2 mal in je 30 ml Toluol suspendiert und erneut die flüchtigen Bestandteile im Vakuum entfernt. Man erhält 570 mg eines blaßgelben Feststoffs, der wegen ungenügender Löslichkeit ohne Reinigung weiter umgesetzt wird.
R_{f}(EE/HEP 2:1) = 0.24

### i) 4'-(5-Formyl-4-methoxy-2-phenyl-imidazol-1-ylmethyl)-3'-methansulfonylbiphenyl-2-sulfonamid

570 mg 4'-(4-Chloro-5-formyl-2-phenyl-imidazol-1-ylmethyl)-3'-methansulfonylbiphenyl-2-sulfonamid sowie 430 mg NaOH werden in 11 ml Methanol 8 Stunden unter Rückfluß gekocht. Das Solvens wird im Vakuum entfernt, der Rückstand in 100 ml einer halbgesättigten wäßrigen NaHCO₃-Lösung suspendiert und 3 mal mit je 100 ml EE extrahiert. Über Na₂SO₄ wird getrocknet das Solvens im Vakuum entfernt und an Kieselgel mit EE/HEP 2:1 chromatographiert. Man erhält 80 mg eines farblosen Öls.
R_{f}(EE/HEP 2:1) = 0.22 MS (ES) : 526 (M+H)⁺

### j) 4'-(5-Formyl-4-methoxy-2-phenyl-imidazol-1-ylmethyl)-3'-methansulfonylbiphenyl-2-sulfonylcyanamid

70 mg 4'-(5-Formyl-4-methoxy-2-phenyl-imidazol-1-ylmethyl)-3'-methansulfonylbiphenyl-2-sulfonamid werden nach der allgemeinen Vorschrift zur Herstellung von Sulfonylcyanamiden aus Sulfonamiden umgesetzt (Reaktionszeit 30 Minuten) und nach Chromatographie an Kieselgel mit EE/MeOH 1:10 erhält man 50 mg weißer Kristalle, mp 210 °C(unter Zersetzung).
R_{f}(EE/MeOH 1:10) = 0.27 IR (C≡N) : 2178.1 cm⁻¹ MS (ES) : 551 (M+H)⁺

### Beispiel 2:

### 4'-(4-Chlor-5-formyl-2-phenyl-imidazol-1-ylmethyl)-3'-methansulfonyl-biphenyl-2-sulfonylcyanamid

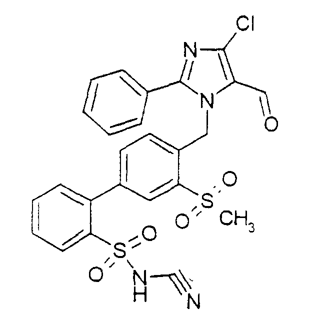

6.5 g 4'-(4-Chlor-5-formyl-2-phenyl-imidazol-1-ylmethyl)-3'-methansulfonyl-biphenyl-2-sulfonamid (Beispiel 1 h) und 5.1 g K₂CO₃ werden in 123 ml wasserfreiem Acetonitril suspendiert und 2.5 ml einer 5N Lösung von BrCN in Acetonitril zugespritzt. 155 Minuten wird unter Rückfluß gekocht und nach Abkühlung das gesamte Reaktionsgemisch an Kieselgel chromatographiert mit EE/MeOH 10:1. Man erhält 5.9 g farbloser Kristalle, mp 220°C (unter Zersetzung).
R_{f}(EE/MeOH 10:1) = 0.18 MS (ES) : 555 (M+H)⁺

### Pharmakologische Daten:

### Inhibition des Na⁺- abhängigen Cl⁻/HCO₃⁻ - Austauschers (NCBE) in humanen Endothelzellen

Humane Endothelzellen (ECV-304) wurden aus Kultur-flaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCl, 20 NH₄Cl, 5 KCl, 1 CaCl₂, 1 MgSO₄, 20 N-(2-Hydroxyethyl)piperazin-N=-2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37°C inkubiert. Anschließend wurden die Zellen gewaschen und in einer Natrium- und Bicarbonatfreien Pufferlösung (mmol/l: 5 HEPES, 133,8 Cholinchlorid, 4,7 KCl, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min).
Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (pHᵢ) über den NCBE, eine Bicarbonat-und Natrium-haltige Pufferlösung (mmol/l: 5 HEPES, 93,8 NaCl, 40 NaHCO₃, 4,7 KCl, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCl, 4,7 KCl, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen wurden in verschiedenen Konzentrationen der Natrium-und Bicarbonat-haltigen Lösung zugesetzt. Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem Well der Mikrotiterplatte bestimmt. Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurde auf die beiden Kontrollen bezogen und daraus die Hemmwirkung der Substanzen ermittelt.

### Ergebnisse

Restaktivität des NCBE bei einer Inhibitorkonzentration von 10 µM (in %)

### Verbindung des Beispiels Nr.

- 1: 14,8
- 2: 18,3

## Patentansprüche

1. Verbindungen der Formel I, 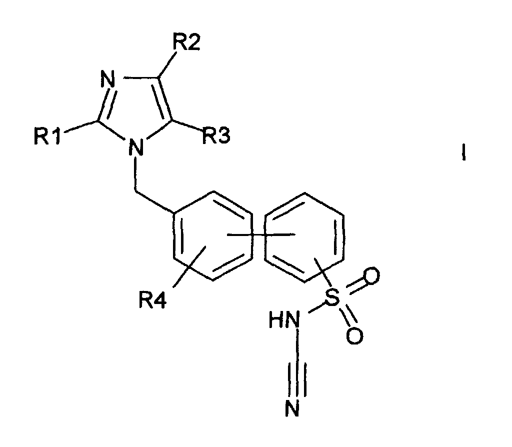 in welcher die Symbole folgende Bedeutung haben:
R(1) -CₐH₂ₐ-Phenyl, wobei der Phenylteil unsubstituiert oder substituiert ist mit 1 oder 2 gleichen oder verschiedenen Resten aus der Reihe F, Cl, Br, CF₃, Methyl, Methoxy, Hydroxy oder NR(8)R(9); R(8) und R(9) unabhängig voneinander
Wasserstoff oder Methyl;
a Null oder 1;
R(2) F, Cl, Br oder I; oder O(R7);
R(7) Alkyl mit 1, 2, 3 oder 4 C-Atomen;
R(3) CO-R(6);
R(6) Wasserstoff;
R(4) SO₂R(16) mit R(16) gleich Alkyl mit 1, 2, 3 oder 4 C-Atomen;
sowie deren physiologisch verträglichen Salze.

2. Verbindungen der Formel I gemäß Anspruch 1, worin R(16) Methyl bedeutet, sowie deren physiologisch verträglichen Salze.

3. Verbindungen der Formel I, in denen die Reste R(1 ), R(2), R(3) und R(4) wie in Anspruch 1 und/oder 2 definiert sind und der Biphenylsubstituent wie in Formel la oder lb verknüpft ist, 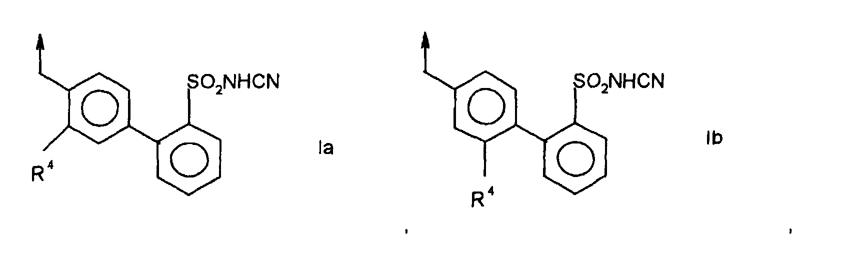 sowie deren physiologisch verträglichen Salze.

4. Verbindungen der Formel I gemäß einem oder mehreren der Ansprüche 1 bis 3, wobei diese
4'-(5-Formyl-4-methoxy-2-phenyl-imidazot-1-ylmethyl)-3'-methansulfonyl-biphenyl-2-sulfonylcyanamid, oder
4'-(4-Chlor-5-formyl-2-phenyl-imidazol-1ylmethyl)-3'-methansulfonyl-biphenyl-2-sulfonylcyanamid
sind, sowie deren physiologisch verträglichen Salze.

5. Verbindungen der Formel I gemäß einem oder mehreren der Ansprüche 1 bis 4 und/oder ihre physiologisch verträglichen Salze zur Anwendung als Arzneimittel.

6. Pharmazeutische Zubereitung, **gekennzeichnet durch** einen wirksamen Gehalt einer Verbindung der Formel I nach einem oder mehreren der Ansprüche 1 bis 4 und/oder eines physiologisch verträglichen Salzes davon.

7. Pharmazeutische Zubereitung gemäß Anspruch 6, **dadurch gekennzeichnet, daß** sie zusätzlich eine wirksame Menge eines NHE-Inhibitors und/oder eine wirksame Substanz aus einer anderen Herz-Kreislauf-Wirkstoffklasse, und/oder deren physiologisch verträglichen Salze enthält.

8. Verbindungen der Formel I gemäß einem oder mehreren der Ansprüche 1 bis 4 und/oder ihre physiologisch verträglichen Salze zur Verwendung als Inhibitor des Natrium-abhängigen Bicarbonat/Chlorid-Austauschers.

9. Verbindungen der Formel I gemäß einem oder mehreren der Ansprüche 1 bis 4 und/oder ihre physiologisch verträglichen Salze zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

10. Verbindungen der Formel I gemäß einem oder mehreren der Ansprüche 1 bis 4 und/oder ihre physiologisch verträglichen Salze zur Verwendung als Antiatherosklerotikum, Mittel gegen diabetische Spätkomplikationen, Krebserkrankungen, fibrotische Erkrankungen oder Organhypertrophien und/oder -hyperplasien.

11. Pharmazeutische Zubereitung gemäß Anspruch 6 und/oder 7 zur Verwendung in der Theraphie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

12. Pharmazeutische Zubereitung gemäß Anspruch 6 und/oder 7 zur Verwendung als Antiatherosklerotikum, Mittel gegen diabetische Spätkomplikationen, Krebserkrankungen, fibrotische Erkrankungen oder Organhypertrophien und/oder-hyperplasien.

## Claims

1. A compound of the formula I 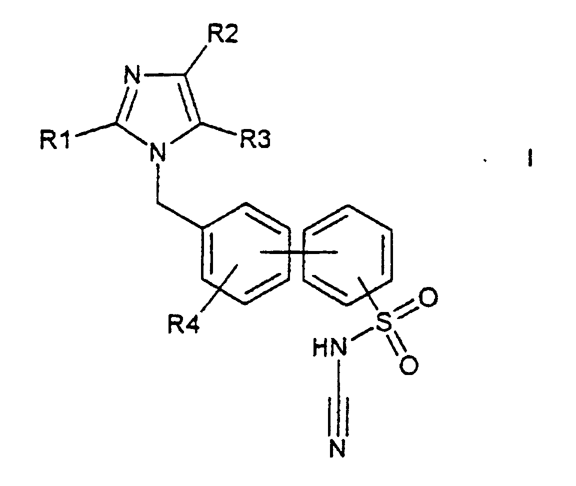 in which the symbols have the following meaning:
R(1) is -CₐH₂ₐ-phenyl, where the phenyl moiety is unsubstituted or substituted by 1 or 2 identical or different radicals from the group consisting of F, Cl, Br, CF₃, methyl, methoxy, hydroxyl or NR(8)R(9);
R(8) and R(9) independently of one another are hydrogen or methyl;
a is zero or 1;
R(2) is F, Cl, Br or I; or O(R7);
R(7) is alkyl having 1, 2, 3 or 4 carbon atoms;
R(3) is CO-R(6);
R(6) is hydrogen;
R(4) is SO₂R(16) where R(16) is alkyl having 1, 2, 3 or 4 carbon atoms;
or its physiologically tolerable salts.

2. A compound of the formula I as claimed in claim 1, where R(16) is methyl, or its physiologically tolerable salts.

3. A compound of the formula I, in which the radicals R(1), R(2), R(3) and R(4) are as defined in claim 1 and/or 2 and the biphenyl substituent is linked as in formula la or lb, 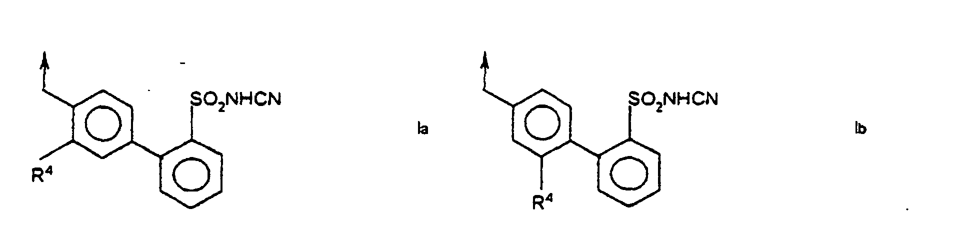 or its physiologically tolerable salts.

4. A compound of the formula I as claimed in one or more of claims 1 to 3, this compound being 4'-(5-formyl-4-methoxy-2-phenylimidazol-1-ylmethyl)-3'-methanesulfonylbiphenyl-2-sulfonylcyanamide or 4'-(4-chloro-5-formyl-2-phenylimidazol-1-ylmethyl)-3'-methanesulfonylbiphenyl-2-sulfonylcyanamide, or its physiologically tolerable salts.

5. A compound of the formula I as claimed in one or more of claims 1 to 4 and/or its physiologically tolerable salts for use as a pharmaceutical.

6. A pharmaceutical preparation which comprises an efficacious amount of a compound of the formula I as claimed in one or more of claims 1 to 4 and/or of a physiologically tolerable salt thereof.

7. The pharmaceutical preparation as claimed in claim 6, which additionally contains an efficacious amount of an NHE inhibitor and/or an active substance from another class of cardiovascular active compound, and/or its physiologically tolerable salts.

8. A compound of the formula I as claimed in one or more of claims 1 to 4 and/or its physiologically tolerable salts for use as an inhibitor of the sodium-dependent bicarbonate/chloride exchanger.

9. A compound of the formula I as claimed in one or more of claims 1 to 4 and/or its physiologically tolerable salts for use in the therapy and/or prophylaxis of cardiac infarct, of angina pectoris, of illnesses caused by ischemic conditions, of disturbed respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

10. A compound of the formula I as claimed in one or more of claims 1 to 4, and/or its physiologically tolerable salts for use as an antiatherosclerotic, agent against diabetic late complications, carcinomatous disorders, fibrotic disorders or organ hypertrophy and/or hyperplasia.

11. A pharmaceutical preparation as claimed in claim 6 and/or 7 for use in the therapy and/or prophylaxis of cardiac infarct, of angina pectoris, of illnesses caused by ischemic conditions, of disturbed respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

12. A pharmaceutical preparation as claimed in claim 6 and/or 7 for use as antiatherosclerotic, agent against diabetic late complications, carcinomatous disorders, fibrotic disorders or organ hypertrophy and/or hyperplasia.

## Revendications

1. Composés de formule I 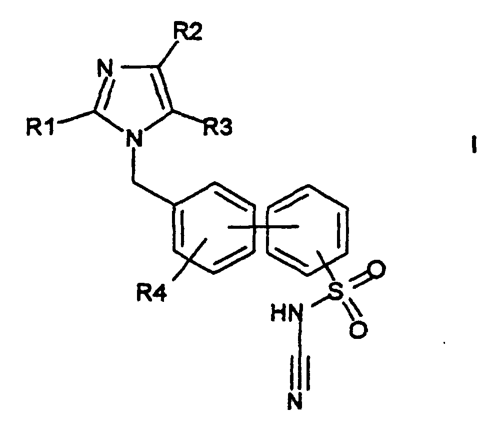 dans lequel les symboles ont la signification suivante:
R(1) représente un groupe -CₐH₂ₐ-phényle, dans lequel la partie phényle est non substituée ou substituée par 1 ou 2 restes identiques ou différents de la série des F, Cl, Br, CF₃, méthyle, méthoxy, hydroxy ou NR(8)R(9);
R(8) et R(9) représentent, indépendamment l'un de l'autre, un atome d'hydrogène ou un groupe méthyle;
a est 0 ou 1;
R(2) est F, Cl, Br ou I; ou OR(7);
R(7) est un groupe alkyle de 1, 2, 3 ou 4 atomes de carbone;
R(3) est un groupe CO-R(6);
R(6) est un atome d'hydrogène;
R(4) est un groupe SO₂R(16) dans lequel R(16) est un groupe alkyle de 1, 2, 3 ou 4 atomes de carbone;
et leurs sels physiologiquement acceptables.

2. Composés de formule I selon la revendication 1, dans lesquels R(16) représente un groupe méthyle, et leurs sels physiologiquement acceptables.

3. Composés de formule I, dans lesquels les restes R(1), R(2), R(3) et R(4) ont la définition donnée dans la revendication 1 et/ou 2 et le substituant biphényle est lié de la manière indiquée dans la formule Ia ou Ib: 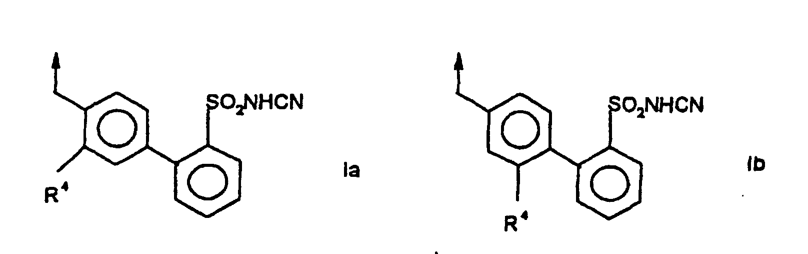 et leurs sels physiologiquement acceptables.

4. Composés de formule I selon l'une ou plusieurs des revendications 1 à 3, qui sont:
le 4'-(5-formyl-4-méthoxy-2-phénylimidazol-1-ylméthyl)-3'-méthanesulfonyl-biphényl-2-sulfonylcyanamide, ou
le 4'-(4-chloro-5-formyl-2-phénylimidazol-1-ylméthyl)-3'-méthanesulfonylbiphényl-2-sulfonylcyanamide,
et leurs sels physiologiquement acceptables.

5. Composés de formule I selon l'une ou plusieurs des revendications 1 à 4 et/ou leurs sels physiologiquement acceptables, à utiliser comme médicaments.

6. Composition pharmaceutique, **caractérisée par** une teneur active en un composé de formule I selon l'une ou plusieurs des revendications 1 à 4 et/ou un de ses sels physiologiquement acceptables.

7. Composition pharmaceutique selon la revendication 6, **caractérisée en ce qu'**elle contient en outre une quantité active d'un inhibiteur de NHE et/ou une substance active d'une autre classe de produits actifs sur le système cardiovasculaire, et/ou leurs sels physiologiquement acceptables.

8. Composés de formule I selon l'une ou plusieurs des revendications 1 à 4 et/ou leurs sels physiologiquement acceptables à utiliser comme inhibiteurs de l'échangeur bicarbonate/chlorure dépendant du sodium.

9. Composés de formule I selon l'une ou plusieurs des revendications 1 à 4 et/ou leurs sels physiologiquement acceptables, à utiliser dans le traitement et/ou la prophylaxie de l'infarctus du myocarde, de l'angine de poitrine, de maladies provoquées par des états ischémiques, de troubles de la respiration, d'états ischémiques du coeur, d'états ischémiques du système nerveux central et périphérique et de l'attaque d'apoplexie, d'états ischémiques d'organes périphériques et des membres, de maladies dans lesquelles la prolifération des cellules représente une cause primaire ou secondaire ou dans le traitement d'états de choc, ou à utiliser dans des opérations chirurgicales et des greffes d'organes ou pour la conservation et le stockage de greffons destinés à des mesures chirurgicales.

10. Composés de formule I selon l'une ou plusieurs des revendications 1 à 4 et/ou leurs sels physiologiquement acceptables, à utiliser comme agents antiathérosclérose, comme agents de lutte contre les complications tardives du diabète, les maladies cancéreuses, les fibroses ou les hypertrophies et/ou hyperplasies d'organes.

11. Composition pharmaceutique selon la revendication 6 et/ou 7, à utiliser dans le traitement et/ou la prophylaxie de l'infarctus du myocarde, de l'angine de poitrine, de maladies provoquées par des états ischémiques, de troubles de la respiration, d'états ischémiques du coeur, d'états ischémiques du système nerveux central et périphérique et de l'attaque d'apoplexie, d'états ischémiques d'organes périphériques et des membres, de maladies dans lesquelles la prolifération des cellules représente une cause primaire ou secondaire ou dans le traitement d'états de choc, ou à utiliser dans des opérations chirurgicales et des greffes d'organes ou pour la conservation et le stockage de greffons destinés à des mesures chirurgicales.

12. Composition pharmaceutique selon la revendication 6 et/ou 7, à utiliser comme agent antiathérosclérose, comme agent de lutte contre les complications tardives du diabète, les maladies cancéreuses, les fibroses ou les hypertrophies et/ou hyperplasies d'organes.
